# EUROPÄISCHE PATENTANMELDUNG

(11) **EP 4 201 918 A1**
(43) Veröffentlichungstag der Anmeldung: **28.06.2023**
(21) Anmeldenummer: 21217297.7
(22) Anmeldetag: 23.12.2021
(51) Int. Cl.: C07C 29/76, C07C 31/20, B01J 2/00

(54) **VERBACKUNGSRESISTENTE NEOPENTYLGLYKOL-PRESSLINGE UND VERFAHREN ZUR HERSTELLUNG VERBACKUNGSRESISTENTER NEOPENTYLGLYKOL-PRESSLINGE**

(71) Anmelder: OQ Chemicals GmbH, 40789 Monheim am Rhein (DE)
(72) Erfinder: LANGE, Horst, 44879 Bochum (DE); ZIMMERER, Julia, 50825 Köln (DE)
(74) Vertreter: Michalski Hüttermann & Partner Patentanwälte mbB

(57) **Zusammenfassung**

Die vorliegende Erfindung betrifft ein Verfahren zur Herstellung von Neopentylglykol-Presslingen, wobei das Verfahren mindestens die Verfahrensschritte umfasst:
a) Bereitstellen einer Schüttung aus NPG-Schuppen;
b) Komprimieren der Schüttung in einer Form zur Ausbildung eines Presslings, wobei das Komprimieren bei einem Druck von größer oder gleich 0,5 MPa und kleiner oder gleich 7,5 MPa erfolgt. Des Weiteren betrifft die vorliegende Erfindung NPG-Presslinge.

## Beschreibung

Die vorliegende Erfindung betrifft ein Verfahren zur Herstellung von Neopentylglykol-Presslingen, wobei das Verfahren mindestens die Verfahrensschritte umfasst:
a) Bereitstellen einer Schüttung aus NPG-Schuppen;
b) Komprimieren der Schüttung in einer Form zur Ausbildung eines Presslings, wobei das Komprimieren bei einem Druck von größer oder gleich 0,5 MPa und kleiner oder gleich 7,5 MPa erfolgt. Des Weiteren betrifft die vorliegende Erfindung NPG-Presslinge.

Neopentylglykol (NPG, 2,2-Dimethylpropan-1,3-diol) ist ein wichtiges Diol, welches beispielsweise in großen Mengen zur Herstellung von Polyestern und Polyurethanen eingesetzt wird. Die industrielle Herstellung des NPGs geht üblicherweise von Isobutylaldehyd aus, welches mit Formaldehyd umgesetzt und das Reaktionsprodukt anschließend katalytisch hydriert wird. Das NPG ist eine hygroskopische, kristalline Verbindung, welche einen Schmelzpunkt von ca. 129°C aufweist. Aus Kosten- und Praktikabilitätsgründen wird das Diol dabei in Gestalt kleinerer Schuppen angeboten, welche durch Verfestigen aus einer NPG-Schmelze mit Hilfe eines Kristallisierungs- oder Kühlbands und anschließendem Brechen in einzelne, mehr oder wenige unregelmäßige Plättchen hergestellt werden. Die NPG-Schuppen werden anschließend zweckmäßigerweise in Bigbags zu größer oder gleich 250kg oder als Sackware zu jeweils größer oder gleich 25 kg konfektioniert und ausgeliefert. Basierend auf den hygroskopischen und komplexen thermodynamischen Phaseneigenschaften des NPGs kann es als Funktion der konkret vorliegenden Lagerungs- und Transportbedingungen dazu kommen, dass die Schuppen im Laufe der Zeit in Form größerer Aggregate zusammenbacken können. Die ungeregelte Agglomeration kann zur Ausbildung sehr großer, kompakter NPG-Klumpen führen, welche die Fähigkeit des Produktes frei zu fließen gegen Null gehen lassen. Dieser Effekt kann soweit gehen, dass der gesamte Inhalt eines Bigbags großvolumig verbackt, sodass eine effiziente Entleerung des Bigbags zur Weiterverarbeitung nicht mehr möglich ist. Zur Verwendung des Produktes in den Standardproduktionsprozessen muss vor der weiteren Verarbeitung eine aufwändige und teure, manuelle Zerkleinerung zum wieder frei fließenden Produkt durchgeführt werden. Dieser ungünstige Zustand führt zu großen Störungen im Produktionsablauf und in der Regel zu Reklamationen an den Hersteller.

Auch in der Patentliteratur finden sich die unterschiedlichen Ansätze zur Konfektionierung und zur Verbesserung der NPG-Verbackungs- bzw. Verpackungsproblematik.

So lehrt beispielsweise die US 4,435,603A den Zusatz von tertiären Aminen in Konzentrationen von 0,25 - 0,5 Gew.% als Antibackmittel bei der Herstellung von Polyol-, insbesondere von Neopentylglykol-Flakes. Allerdings lehrt die Erfahrung, dass auch der Zusatz derartiger Antibackmittel das Entstehen von Materialverbackungen, grober Knollen und Klumpen wie auch großvolumige Produktverbackungen, gerade bei der Lagerung von palettierten Säcken oder Bigbags, nicht zuverlässig verhindern.

So beschreibt beispielsweise die DE 3 522 359 A1 ein Verfahren zum Konfektionieren von unter Normalbedingungen kristallinen, organischen Materialien, bei dem die Materialien in pulverförmigem und/oder schmelzflüssigem Zustand in einer selbstreinigenden, zweiwelligen Schneckenmaschine mit im gleichen Sinne rotierenden Schneckenwellen aufbereitet, durch wenigstens einen verengten Durchgang in eine Zone von niederem Druck ausgestoßen, gekühlt und in Teilchen zerkleinert werden, dadurch gekennzeichnet, dass die Materialien während des Austragens durch den verengten Durchgang zur Bildung eines Schmelzefilms erwärmt werden.

Des Weiteren offenbart die EP 0 829 298 A2 ein Verfahren zur Herstellung von Hydroxypivalinsäureneopentylglykolester-Granulaten durch Aufbringen einer Hydroxypivalinsäureneopentylglykolester-Schmelze auf eine Kühlfläche, auf der die Schmelze erstarrt, dadurch gekennzeichnet, dass die Schmelze mindestens 3 Gew-%, bezogen auf die Gesamtmenge an Hydroxypivalinsäureneopentylglykolester, an Hydroxypivalinsäureneopentylglykolester-Kristallen enthält.

Auch in der EP 1 268 378 B1 wird ein Verfahren zur Konfektionierung von Neopentylglykol durch Abkühlen, Kristallisieren und Zerkleinern einer Neopentylglykol-Schmelze und nachfolgendes Verpacken der so erhaltenen Neopentylglykol-Teilchen in Lager- oder Transportbehältnisse beschrieben. In diesem Verfahren wird die Schmelze zu Beginn des Abkühlens für mindestens 1/10 Minute ohne Verwendung eines Kühlmittels oder unter Verwendung eines Kühlmittels mit einer Temperatur im Bereich von 50 bis 120°C gekühlt und mit einer Temperatur unterhalb von 30°C verpackt.

Derartige aus dem Stand der Technik bekannte Lösungen können noch weiteres Verbesserungspotential bieten. Dies bezieht sich insbesondere auf die Bereitstellung möglichst hochreiner NPG-Formkörper, welche auch unter ungünstigen Lagerbedingungen ein nur geringes Maß an Verbackungsneigung zeigen.

Es ist die Aufgabe der vorliegenden Erfindung, ein verbessertes Verfahren zur Gewinnung verbackungsresistenterer NPG-Formkörper in Form von Presslingen, sowie eine verbesserte Darreichungsform des NPGs mit einer verringerten Neigung zur Verklumpung während der Lagerung bereitzustellen.

Die Lösung der Aufgabe erfolgt durch die Merkmale der unabhängigen Ansprüche, gerichtet auf das erfindungsgemäße Verfahren sowie die erfindungsgemäßen Presslinge. Bevorzugte Ausgestaltungen der Erfindung sind in den Unteransprüchen, in der Beschreibung oder den Figuren angegeben, wobei weitere in den Unteransprüchen, in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Erfindungsgemäß gelöst wird die Aufgabe durch ein Verfahren zur Herstellung von Neopentylglykol-Presslingen, wobei das Verfahren mindestens die Verfahrensschritte a) Bereitstellen einer Schüttung aus NPG-Schuppen; und b) Komprimieren der Schüttung in einer Form zur Ausbildung eines Presslings, wobei das Komprimieren bei einem Druck von größer oder gleich 0,5 MPa und kleiner oder gleich 7,5 MPa erfolgt, umfasst. Überraschenderweise wurde gefunden, dass sich über das erfindungsgemäße Verfahren eine Vielzahl unterschiedlicher und mechanisch stabiler NPG-Presslinge erhalten lassen, welche im Rahmen einer Lagerung oder eines Transportvorganges im Vergleich zu NPG-Schuppen eine deutlich verringerte Neigung zur Ausbildung größerer Aggregate zeigen. Zusätzlich zu einer geringeren Verbackungsneigung unter unterschiedlichen Umgebungsbedingungen, zeigen die mittels des Verfahrens herstellbaren Presslinge zudem eine geringere Sublimationsneigung und, besonders überraschend, für eine großtechnische Verarbeitung nur unwesentlich eingeschränkte Auflösbarkeit in unterschiedlichen Lösungsmitteln. Ohne durch die Theorie gebunden zu sein ergeben sich die synergistischen Vorteile aus hoher mechanischer Belastbarkeit, geringer Sublimationsneigung, kleinerer Verbackungsneigung bei einem relativ gutem Auflösevermögen durch den Einsatz von NPG-Schuppen innerhalb eines mechanischen Pressvorgangs mit relativ geringen Drücken, wobei ein mechanisch sehr stabiler aber dennoch durch Lösemittel leicht in seine Bestandteile auflösbarer Formkörper erhalten wird. Durch die Verwendung von Schuppen scheinen wohl, bedingt durch die Größenverteilung der Schuppen und deren unregelmäßige Gestalt, im Pressvorgang im angegebenen Druckbereich das richtige Maß an Hohlräumen im Pressling eingeschlossen zu werden, welche die mechanische Festigkeit des Presslings nur gering, die Auflösegeschwindigkeit hingegen aber sehr positiv beeinflussen. Höhere Druckbereiche beim Pressen führen eher dazu, dass die mechanische Festigkeit nur unwesentlich erhöht, die Auflösegeschwindigkeit in Lösemitteln aber stark reduziert wird. Letzteres wahrscheinlich bedingt durch eine zu starke Erhöhung der Pressling-Dichte, welche ein Eindringen von Lösemitteln in den Pressling erschwert. Ein geringerer Pressdruck kann eher dazu führen, dass die mechanische Festigkeit des Presslings für übliche Lager- und Transportbedingungen nur unzureichend ist. Zudem ist weiterhin erstaunlich, dass der erfindungsgemäß vorgeschlagene Pressvorgang auch die gewünschte NPG-Kristallstruktur nicht ändert oder einschränkt, sodass keine oder nur sehr kleine Wärmetönungen im Press- und im anschließenden Lagervorgang auftreten, welche die grundlegenden Eigenschaften des Presslings unverändert lassen. Dies resultiert im Gegensatz zu den im Stand der Technik ansonsten vorgeschlagenen Lösungen in einem deutlichen Vorteil, da die Temperaturführung im Pressvorgang einfacher gehalten werden kann. Schließlich und endlich ist besonders vorteilhaft, dass diese Eigenschaftsverbesserungen ohne die Zusätze weiterer Substanzen im NPG-Pressling realisiert werden können. Letztere Lösung ist besonders nachteilig, da diese weiteren Substanzen im weiteren Verfahrensverlauf entweder aufwendig entfernt oder im Rahmen der Rezepturierung weiterer Folgeprodukte mit ihren Eigenschaften berücksichtigt werden müssen oder sich negativ auf die Qualität der daraus hergestellten Folgeprodukte auswirken können.

Das erfindungsgemäße Verfahren ist ein Verfahren zur Herstellung von Neopentylglykol-Presslingen. Die Presslinge sind dabei Formkörper, welche durch das Zusammenpressen von Material in einer Pressform erhalten werden. Die Presslinge zeichnen sich dabei durch eine regelmäßige Form aus, welche sich aus der Geometrie der verwendeten Pressform ergibt. So können beispielsweise über die Wahl der Pressform Kugeln, Quader oder Briketts in unterschiedlichen Ausgestaltungen und Größen hergestellt werden. Die Presslinge sind im Wesentlichen gleich ausgeformt, so dass beispielsweise unterschiedliche Presslinge sich in ihrem Gewicht um weniger als 25 Gew.-%, bevorzugt weniger als 15 Gew.-% und des Weiteren bevorzugt um weniger als 10 Gew.-% unterscheiden. Der Pressling ist ein Neopentylglykol-Pressling in den Fällen, in denen mehr als 90 Gew.-%, des Weiteren mehr als 95 Gew.-%, und weiter bevorzugt mehr als 97 Gew.-% des Presslings aus NPG bestehen. Das Verfahren kann mittels üblicher Pressen in Form von Extruderpressen, Walzenpressen oder im Presskammerverfahren durchgeführt werden.

Das Verfahren umfasst den Verfahrensschritt a), in welchem das Bereitstellen einer Schüttung aus NPG-Schuppen erfolgt. Der NPG-Pressling wird aus einer Schüttung von NPG-Schuppen hergestellt. Bei dieser Schüttung handelt es sich dabei um eine statistische Verteilung unregelmäßig gebrochener NPG-Teilchen. Die NPG-Schuppen weisen eine Dicken- und Größenverteilung auf, welche eine Funktion des Herstellungsprozesses ist. Neben den makroskopischen Schuppen können in der Schüttung auch NPG-Teilchen in Form von kleinen Körnern oder NPG-Staub vorliegen. Der Staubanteil der NPG-Schüttung ist dabei ebenfalls eine Funktion des Herstellungsprozesses. Mengenmäßig liegen in der Schüttung überwiegend Schuppen in Form flacher Plättchen vor, welche einen unregelmäßig gebrochenen Rand aufweisen. Die Größenverteilung der NPG-Schuppen kann über das mechanische Einwirken auf das auf einem Kühlband erstarrte NPG bestimmt werden. Die Zusammensetzung der NPG Schuppen kann dabei in gewissen Anteilen frei gewählt werden. Bevorzugt kann die NPG-Schüttung aus reinem NPG bestehen. Es ist aber auch möglich, dass die Schuppen als weitere Bestandteile noch andere Substanzen aufweisen. Bevorzugt beträgt der Gewichtsanteil des NPG in den Schuppen größer oder gleich 80 %, des Weiteren bevorzugt größer oder gleich 90 % und weiterhin bevorzugt größer oder gleich 95 %.

Das Verfahren umfasst den Verfahrensschritt b), in welchem das Komprimieren der Schüttung in einer Form zur Ausbildung eines Presslings erfolgt. Erfindungsgemäß erfolgt das Komprimieren bei einem Druck von größer oder gleich 0,5 MPa und kleiner oder gleich 7,5 MPa. Die in eine oder mehrere Pressformen gefüllte Schüttung aus NPG-Schuppen wird durch Kontaktieren der beiden Pressformhälften dem oben genannten Druckbereich ausgesetzt. Dieser Vorgang kann beispielsweise durch eine manuelle Presse oder aber kontinuierlich durch umlaufende Presswerkzeuge erfolgen. Der Pressvorgang kann wahlweise innerhalb einer konditionierten Umgebung stattfinden. Es ist aber nicht zwingend, dass die Umgebungsluft oder die -temperatur entsprechend vorkonditioniert werden. Zweckmäßigerweise kann der Pressvorgang bei Raumtemperatur erfolgen. Es ist aber auch möglich, dass beispielsweise die Presswerkzeuge auf einen bestimmten Temperaturbereich, beispielsweise zwischen größer oder gleich 10°C und kleiner oder gleich 40°C vortemperiert werden. Die Zeitspanne für das Pressen einer einzelnen Form kann variieren. So kann die Schüttung aus NPG-Schuppen beispielsweise für größer oder gleich 1 Sekunde, weiter bevorzugt größer oder gleich 2 und des Weiteren bevorzugt größer oder gleich 5 Sekunden im Presswerkzeug verbleiben. Die Anwendung des Pressdruck über eine längere Zeitdauer ist dabei unnötig. Im Rahmen der Pressung können neben den einzelnen Presslingen auch noch Verbindungsstücke zwischen den Presslingen ausgebildet werden, welche sich dadurch ergeben, dass zwischen den einzelnen Pressformen ebenfalls Material aus der Schüttung abgelegt wurde. Dieses weitere Material bildet nicht die Presslinge im Sinne der Erfindung und kann vor der weiteren Verarbeitung der Presslinge beispielsweise durch geringen mechanischen Druck abgeschert oder abgesiebt werden.

In einer bevorzugten Ausführungsform des Verfahrens können die NPG-Schuppen eine Schüttdichte von größer oder gleich 0,5 g/cm³ und kleiner oder gleich 0,6 g/cm³ aufweisen. Zur Ausbildung besonders mechanisch stabiler und insbesondere besonders gut auflösbarer Presslinge hat sich als besonders vorteilhaft herausgestellt, dass die Schüttdichte der NPG-Schüttung im oben angegebenen Bereich liegt. Ohne durch die Theorie gebunden zu sein scheint insbesondere auch die Schüttdichte für die bevorzugte Porosität der Presslinge ausschlaggebend zu sein. Kleinere Schüttdichten können nachteilig sein, da in diesen Fällen die mechanische Festigkeit der Presslinge ungenügend sein kann. Größere Schüttdichten können hingegen nachteilig sein, da in diesen Fällen die Auflösegeschwindigkeit des Presslings zu stark herabgesetzt wird. Die Messung der Schüttdichte kann beispielsweise nach DIN ISO 697 oder DIN ISO 60 durchgeführt werden.

Innerhalb einer weiter bevorzugten Ausgestaltung des Verfahrens können die NPG-Schuppen einen Feinanteil von kleiner oder gleich 6 mm, ermittelt durch Siebung, von größer oder gleich 80 Gew.-% und kleiner oder gleich 90 Gew.-% aufweisen. Für die mechanischen Eigenschaften des Presslings hat sich zudem als vorteilhaft herausgestellt, dass die eingesetzten NPG-Schuppen der NPG-Schüttung einer bestimmten Größenverteilung genügen. Insbesondere ein höherer Anteil an kleinen Schuppen, hier angegeben als Feinanteil mit einer Größe kleiner als 6 mm, kann dazu führen, dass sowohl die mechanische Festigkeit wie auch das Auflösevermögen des Presslings verbessert werden. Innerhalb eines weiter bevorzugten Aspektes des Verfahrens können die NPG-Schuppen eine Dicke von größer oder gleich 0,75 mm und kleiner oder gleich 5 mm aufweisen. Neben der lateralen Ausdehnung der NPG Schuppen hat auch deren Dicke einen Einfluss auf die erreichbare Festigkeit und die erreichbare Auflösegeschwindigkeit von NPG-Presslingen. Die Dicke der Schuppen kann dabei zweckmäßigerweise durch die Höhe der NPG-Schmelze auf dem zur Herstellung benutzen Kühlband bestimmt werden. Kleinere Dicken der NPG-Schuppen können nachteilig sein, da in diesen Fällen die mechanische Festigkeit des erhältlichen Presslings herabgesetzt sein kann. Höhere Dicken können nachteilig sein, da in diesen Fällen ebenfalls ungünstige mechanische Eigenschaften des Presslings erhalten werden können. Ohne durch die Theorie gebunden sein kann dieser Zusammenhang wahrscheinlich darin liegen, dass die einzelnen Schuppen einer Verformung in der Presse stärker widerstehen können und insofern eine verringerte Wechselwirkung zwischen den einzelnen Schuppen durch den Pressvorgang induziert wird. Die Dicke der NPG-Schuppen kann beispielsweise mit einer Schieblehre bestimmt werden. Zum Erhalt eines statistisch abgesicherten Wertes über die Schüttung können beispielsweise 50 ausgewählte Schuppen vermessen werden. Weiterhin vorteilhaft kann die Dicke der Schuppen größer oder gleich 1 mm und kleiner oder gleich 4 mm, des Weiteren bevorzugt größer oder gleich 1,5 mm und kleiner oder gleich 3 mm betragen.

Nach einer bevorzugten Charakteristik des Verfahrens können die NPG-Schuppen ein D50-Quantil, ermittelt über Siebung, von größer oder gleich 2 mm und kleiner oder gleich 8 mm aufweisen. Zur Herstellung besonders mechanisch stabiler und sich schnell auflösender Presslinge haben sich NPG-Schuppen mit oben angegebenen Quantil als besonders geeignet herausgestellt. Dieser Größenbereich der NPG-Schuppen mit einem hohem Anteil eher kleinerer Teilchen kann zusammen mit einen relativ geringen Pressdruck und einer relativ kurzen Presszeit dazu führen, dass besonders geeignete Wechselwirkungen zwischen den einzelnen zu verpressenden Teilchen hergestellt werden. Es bildet sich ein sehr homogener Pressling, welcher sich bei Kontakt mit einem Lösemittel in relativ kurzer Zeit gut auflöst.

In einer weiter bevorzugten Ausführungsform des Verfahrens können die NPG-Schuppen ein D95-Quantil, ermittelt über Siebung, von größer oder gleich 7,5 mm und kleiner oder gleich 10 mm aufweisen. Auch kann der Einsatz von NPG-Schuppen mit einem relativ geringen Anteil an Schuppen über 10 mm dazu führen, dass besonders homogene Presslinge erhältlich sind, welche auch bei stärkerer mechanischer Belastung sich durch nur einen geringen Anteil an Bruchstücken auszeichnen.

In einer weiter bevorzugten Ausführungsform des Verfahrens kann das Komprimieren bei einem Druck von größer oder gleich 1,0 MPa und kleiner oder gleich 4 MPa erfolgen. Zum erhält möglichst gleichmäßiger und mechanisch stabiler Presslinge hat sich oben angegebener Pressdruck als besonders geeignet herausgestellt. Insbesondere in dem Bereich doch relativ geringer Pressdrücke kann eine ausreichende Stabilität und ein sehr schnelles Auflöseverhalten der Presslinge erreicht werden.

In einer weiteren Ausgestaltung des Verfahrens kann der Verfahrensschritt b) in einem Temperaturbereich von größer oder gleich 5°C und kleiner oder gleich 40°C erfolgen. Zum Erhalt besonders gleichmäßiger Presslinge und zur Verhinderung von Anbackungen in den Pressformen hat sich oben angegebener Temperaturbereich als besonders geeignet herausgestellt. Höhere Temperaturen können nachteilig sein, da in diesen Bereichen ungewollte thermodynamische Phasenumwandlung des NPGs induziert werden können. Des Weiteren können höhere Temperaturen im Verfahren dazu führen, dass sich im Laufe einer kontinuierlichen Herstellung ungewollt Produktionsmaterial an den Wandungen der Presse festsetzt, welches im Rahmen der Pressvorgänge nicht eigenständig aus der Form entfernt wird. Tiefere Temperaturen beim Pressen können nachteilig sein, da in diesen Fällen durch den geringeren Pressdruck eine nur unzureichende Verpressung der einzelnen Teilchen untereinander stattfindet.

Innerhalb einer weiter bevorzugten Ausgestaltung des Verfahrens können die NPG-Schuppen einen Wassergehalt von größer oder gleich 0,05 Gew.-% und kleiner oder gleich 3 Gew.-% aufweisen. Zur Herstellung besonders gleichmäßiger Presslinge hat sich als besonders vorteilhaft herausgestellt, dass die NPG-Schuppen einen definierten Wassergehalt aufweisen. Neben dem Einfluss auf die mechanischen Eigenschaften kann der Wassergehalt der Schüttung auch einen Einfluss auf die Produzierbarkeit der Presslinge ausüben. So kann es insbesondere vorkommen, dass mit zu hohen Wasseranteilen die mechanischen Pressformen häufiger gereinigt werden müssen. Der Wassergehalt der NPG Schuppen lässt sich über bekannte Methoden, wie beispielsweise Karl-Fischer, bestimmen.

Des Weiteren erfindungsgemäß ist ein NPG-Pressling, wobei der Pressling eine Dichte von größer oder gleich 0,9 g/cm³ und kleiner oder gleich 1,02 g/cm³ aufweist. Mittels des erfindungsgemäßen Verfahrens lassen sich Presslinge erhalten, welche ausgehend vom eingesetzten NPG und ausgehend von dem Einsatz als NPG-Schuppen einen sehr kleinen Bereich der spezifischen Dichte aufweisen. Innerhalb dieses doch sehr engen NPG-Dichtebereiches ergeben sich mechanisch sehr stabile Presslinge, welche auch unter ungünstigen Lagerbedingungen eine nur sehr geringe Neigung zur Verbackung zeigen. Neben den verbesserten mechanischen Eigenschaften und der geringen Sublimationsneigung der Presslinge als solche, zeigen die Presslinge aber trotzdem eine gute Löslichkeit in den üblichen Lösungsmitteln für NPG, wie beispielsweise Wasser. Diese doch gute Löslichkeit im Vergleich mit NPG-Schuppen ist erstaunlich, da die Presslinge im Vergleich zu den NPG-Schuppen eine deutlich geringere Oberfläche aufweisen. Insofern würde aufgrund des Unterschieds in der Oberfläche der Fachmann erwarten, dass die Auflösegeschwindigkeit der NPG-Presslinge deutlicher herabgesetzt ist. Die Dichte der Presslinge kann über dem Fachmann bekannte Verfahren, beispielsweise durch ausmessen und abwiegen der Presslinge bestimmt werden.

Nach einer bevorzugten Charakteristik des NPG-Presslings kann der Pressling ein Volumen von größer oder gleich 2,5 cm³ und kleiner oder gleich 15 cm³ aufweisen. Zum Erhalt eines möglichst ausgeglichenen Verhältnis zwischen Auflösegeschwindigkeit der einzelnen Teilchen und mechanischer Stabilität des Presslings, haben sich oben angegebene Volumina für den einzelnen Pressling als besonders geeignet herausgestellt. Kleinere Presslinge können eine zu große Oberfläche aufweisen, welches im Rahmen der Lagerung durch eine verstärkte Sublimation von den Oberflächen der einzelnen Pressling zu einem zu hohen Feinanteil führen kann. Größere Pressling-Volumina können hingegen nachteilig sein, da in diesen Fällen die Auflösegeschwindigkeit der Presslinge, beispielsweise in Wasser, zu stark herabgesetzt wird.

In einer weiter bevorzugten Ausführungsform des NPG-Presslings kann der Pressling eine Dichte von größer oder gleich 0,95 g/cm³ und kleiner oder gleich 1,05 g/cm³ aufweisen. Bedingt durch die Schüttdichte der NPG Schuppen und durch den in der Herstellung angewandten Pressdruck, kann die Dichte der resultierenden NPG-Presslinge bestimmt werden. Durch diese Parameter lassen sich insbesondere Presslinge erhalten, welche eine Dichte im oben angegebenen Bereich aufweisen. Dieser doch sehr enge Bereich an Materialdichte für die NPG-Presslinge kann insbesondere dazu führen, dass mechanisch sehr stabile Presslinge erhalten werden, welche eine besonders schnelle Auflösung in den dafür geeigneten Lösemitteln zeigen. Zudem kann dieser Dichtebereich dazu führen, dass die finale mechanische Festigkeit der Presslinge schon nach relativ kurzer Zeit erhalten wird.

Innerhalb eines bevorzugten Aspektes des NPG-Presslings kann der Pressling ein Oberflächen-zu-Massen-Verhältnis von größer oder gleich 0,4 m²/kg und kleiner oder gleich 0,6 m²/kg aufweisen. Insbesondere für NPG-Presslinge hat es sich als geeignet herausgestellt, dass Pressformen mit einer Grundgeometrie eingesetzt werden, welche oben angegebenes Oberflächen-zu-Massenverhältnis aufweisen. Diese Geometrie zeigen ein nur geringes Maß an Masseverlust (Sublimation) während der Lagerung, führen auch bei starker mechanischer Belastung zu nur geringen Abrieb an den einzelnen Presslingen und diese Formen können auch mit einer Standardausrüstung im industriellen Umfeld verarbeitet werden. Das Oberflächen-zu-Massenverhältnis kann über ein Auswiegen und die Bestimmung der Presslinggröße, beispielsweise über eine Schieblehre, bestimmt werden.

In einer weiter bevorzugten Ausführungsform des NPG-Presslings kann der Pressling einen NPG-Gehalt von größer oder gleich 98 Gew-% aufweisen. Überraschenderweise hat sich gezeigt, dass auch ohne den Zusatz weiterer Substanzen, beispielsweise in Form von Sprengmitteln oder in Form von Anti-Verbackungsmitteln, mechanisch stabile Formkörper erhalten werden können, welche eine nur sehr geringe Neigung zur Verbackung während der Lagerung oder des Transports zeigen. Insofern ist besonders hervorzuheben, dass die weitere Verarbeitung des NPGs ohne Rücksicht auf das Vorhandensein weiterer Substanzen durchgeführt werden kann. Insbesondere sind die erfindungsgemäßen Presslinge weitestgehend frei von weiteren Substanzen. So kann der NPG-Anteil in den Pressling größer oder gleich 98,5 Gew.-%, des Weiteren bevorzugt größer oder gleich 99,5 Gew.-% und weiterhin bevorzugt größer oder gleich 99,9 Gew.-% betragen. Besonders bevorzugt kann der Pressling auch zu 100 % aus NPG bestehen. Der Gewichtsanteil des NPGs an den Presslingen lässt sich beispielsweise über eine HPLC Untersuchung unter Vernachlässigung des Wasseranteils quantitativ bestimmen.

In einer weiteren Ausgestaltung des NPG-Presslings kann der Pressling eine Druckfestigkeit von größer oder gleich 80 N und kleiner oder gleich 400 N aufweisen. Die erfindungsgemäßen Presslinge zeichnen sich durch eine relativ hohe Bruchfestigkeit aus, welche erstaunlicherweise über einen nur relativ geringen Pressdruck im Rahmen der Herstellung erhältlich ist. Zum Erhalt konsistenter Werte wird die Bruchfestigkeit der einzelnen Presslinge 24 Stunden nach der Herstellung und Lagerung bei Raumtemperatur bestimmt. Innerhalb dieser Zeitspanne kann der Pressling als solcher noch Nachhärten und höhere Bruchfestigkeiten ausbilden. Die Druckfestigkeit wird dabei mittels eines Druckfestigkeitstesters ("compression strength") der Firma Erichson (Model 469 E4) ermittelt. Die obere und untere Platte weisen einen Durchmesser von jeweils 80 mm auf. Der Durchmesser des Messkörpers beträgt 10 mm (horizontal) und die Geschwindigkeit des Messkörpers beträgt 8mm/min.

Innerhalb einer weiter bevorzugten Ausgestaltung des NPG-Presslings kann der Pressling eine quaderförmige Geometrie aufweisen. Zur Handhabung in industriellen Verpackungs- und Transportprozessen hat sich als besonders geeignet herausgestellt, dass die Presslinge eine quaderförmige Geometrie aufweisen. Diese Geometrie kann zu einer besonders geringeren Verbackungsneigung und zu einem besonders geringen Teil an Abrieb, auch bei starker mechanischer Belastung während des Transports, beitragen. Der Pressling kann dabei eine exakte quaderförmige Geometrie oder aber auch eine Geometrie aufweisen, welche an einen Quader angelehnt ist. So werden über diese Definition Briketts im üblichen Sinne wie auch Eierkohlen oder Eierbriketts verstanden, die eine quaderförmige Grundgeometrie mit abgerundeten Ecken aufweisen. Die quaderförmige Geometrie kann auch ein Kubus sein. Die Presslinge können neben der Grundgeometrie auch noch eine, beispielsweise umlaufende, Pressnaht und weitere Merkmale, wie beispielsweise ein Logo oder ähnliches an der Oberfläche aufweisen.

Innerhalb eines weiter bevorzugten Aspektes des NPG-Presslings kann das Verhältnis aus der gemittelten Länge und Breite zur Höhe des quaderförmigen Presslings, ermittelt nach (Länge + Breite)/ 2 dividiert durch die Höhe, größer oder gleich 1,25 und kleiner oder gleich 3,5 betragen. Aufgrund der Tatsache, dass die Presslinge im Rahmen der industriellen Herstellung nicht ordentlich geschichtet, sondern in einer ungeordneten Schüttung in Beutel oder Big-Bags bereitgestellt werden, hat sich oben angegebenes Verhältnis aus Höhe und Breite des quaderförmigen Presslings als besonders geeignet herausgestellt. Dieses Aspektverhältnis des Presslings kann dazu führen, dass auch unter ungünstigen Lagerbedingungen und unter hoher mechanischer Belastung nur ein geringer Anteil an Bruch in der Pressling-Schüttung erhalten wird. Zudem kann durch die angegebene Asymmetrie des quaderförmigen Presslings auch eine geeignete Auflösegeschwindigkeit des Presslings in den üblichen Lösungsmitteln erreicht werden.

Des Weiteren erfindungsgemäß sind Schüttungen aus verbackungsresistenten Neopentylglykolpresslingen, wobei der Anteil an Feinpartikeln < 1 mm in den Schüttungen unterhalb von 10% liegt. Diese Schüttungen können sich durch einen besonders geringen Anteil an Verbackungen auch unter ungünstigen Lager- und Transportbedingungen auszeichnen.

### Beispiele

Zur Herstellung von NPG-Presslingen wird eine Schüttung aus NPG-Schuppen in einer Walzenpresse zu Presslingen unterschiedlicher Größe in Form von NPG-Briketts komprimiert. Die NPG-Schuppen bestehen (unter Vernachlässigung des Wasseranteils) zu 100% aus NPG. Es werden keine zusätzlichen Anti-Verbackungs-, Binde- oder Sprengmittel den NPG-Schuppen oder dem NPG als solchem zugesetzt. Die Schuppen wurden mittels einer Siebanalyse mit folgenden Größenklassen untersucht (Angaben in mm): > 20, 20-10; 10-8; 8-6,3; 6,3-5; 5-4; 4-3,15; 3,15-2; 2-1; 1-0,5; 0,5-0,25; 0,25-0,125 und 0,125-0 mm. Es ergibt sich ein D50 Quantil von 3,45 mm und ein D95-Quantil von 8,7 mm. Die Dichte der NPG-Schüttung beträgt 0,525 g/cm³.

Es werden zwei unterschiedliche Brikett-Größen hergestellt, wobei die Grundsymmetrie der Pressformen einer Eierkohlen-Brikett-Grundform entspricht. Die Abmessungen der Briketts ergeben sich wie folgt:

| Nennvolumen [cm³] | Länge [mm] | Breite [mm] | Höhe [mm] |
|---|---|---|---|
| 5 | 30 | 24 | 17 |
| 10 | 33 | 30 | 20 |

Das Verpressen wird für beide Größen jeweils bei einem Pressdruck von 1 MPa durchgeführt. Die Briketts werden anschließend zur Abtrennung des Feinanteils (Teilchen kleiner als 6,3 mm) gesiebt. Ohne Feinanteil liegt die Ausbeute des Pressvorgangs bei über 90%. Die Briketts werden für 24 h bei Raumtemperatur und einer relativen Luftfeuchte von 85 % gelagert. Unter diesen Lagerbedingungen wurde keine Veränderung der Brikett-Masse durch hygroskopisches Verhalten beobachtet. Die Druckfestigkeit der Presslinge verbessert sich durch die 24 h Lagerung signifikant. Direkt nach der Verpressung liegt die Druckfestigkeit ermittelt durch eine Druckfestigkeitsmessung ("Compression Strength of Briquettes", gemessen mit einer Maschine Type 469 ERICHSEN) bei >100 N. Nach einer Lagerung von 24 h unter oben angegebenen Bedingungen kann die Druckfestigkeit noch einmal gesteigert werden. Typische Druckfestigkeitswerte liegen nach Lagerung für das kleinere Brikett bei 109 N und für die größeren Briketts bei 155 N. Die Briketts bestehen auch einen Falltest aus 2 m Höhe. Nach 2 Durchläufen an ungelagerten Briketts konnten für beide Brikettgrößen noch ca. 90% intakte Briketts festgestellt werden. Diese Ergebnisse der Falltests verbesserten sich ebenfalls nach einer 24h Lagerung der frisch herstellten Presslinge.

Zur Bestimmung des Masseverlustes der Presslinge durch Sublimation während der Lagerung werden die Briketts in einem Abzug gestellt und der Masseverlust über 49 Tage verfolgt. Die Briketts zeigen ein lineares Sublimationsverhalten, wobei der Masseverlust nach 49 Tagen ca. 10% für den 10cm³- und 11% für den 5cm³-Pressling beträgt. Im Gegensatz dazu beträgt der Masseverlust von NPG-Schuppen unter diesen Bedingungen ca. 18%.

Es werden außerdem Versuche bzgl. der Pressling-Hygroskopie unter definierter Luftfeuchtigkeit in einem Exsikkator durchgeführt. Dazu werden die Briketts in einer offenen Schale in einen mit verschiedenen gesättigten Salzlösungen versehenen Exsikkator gestellt. Gesättigte NaCI-Lösung liefert eine relative Luftfeuchtigkeit von 74%, wohingegen durch die Verwendung gesättigter LiCI-Lösung eine geringere Luftfeuchtigkeit von 11% erzielt wird. Das Gewicht und der Wassergehalt der Presslinge werden nach zwei Wochen notiert. Durch Lagerung bei einer relativen Luftfeuchtigkeit von 11% kann kein signifikanter Anstieg des Wassergehalts oder Masseverlust der Presslinge beobachtet werden. Bei einer Lagerung unter 74% Luftfeuchtigkeit wurde nach 2 Wochen ein Wassergehalt von 1,3% (10cm³ Briketts) bzw. 1,6% (5cm³-Pressling) gemessen werden. Somit zeigen die Presslinge im Vergleich zu NPG-Schuppen eine deutlich geringere Hygroskopie.

Es wurden zudem Lagerversuche an den Presslingen unter Druck durchgeführt. Dazu wird jeweils ein Glaszylinder (Durchmesser 15,3 cm) mit einem Autoklaveneinsatz als Gewicht (14,3 kg) mit einer passenden Teflonscheibe dazwischen verwendet. Dieser Aufbau liefert einem Lagerdruck von insgesamt 78 g/cm² und entspricht in etwa dem Druck, welcher im untersten Sack eines Sackstapels oder im unteren Bereich eines Big-Bags herrscht. Die Versuche werden jeweils mit einer Schütthöhe von 10 cm bei Raumtemperatur durchgeführt. Es kann ein eindeutiger Unterschied des Lagerverhaltens der Presslinge im Vergleich zu den NPG-Schuppen beobachtet werden. Nach einer Woche Lagerung ist im Falle der Presslinge nur ein minimaler Anteil des Materials an der Oberfläche verbacken. NPG-Schuppen zeigen unter diesen Bedingungen einen deutlich höheren Anteil an Verbackungen und Agglomerationen. Selbst anhaftende Briketts lassen sich durch die Anwendungen geringer Kräfte sehr leicht wieder voneinander trennen. Nach einer 4-wöchigen Lagerung ergibt sich für die Presslinge im Vergleich zu den NPG-Schuppen ein ähnliches Bild. Der Grad der Anhaftungen erhöht sich sowohl für die NPG-Schuppen als auch für die Presslinge, dennoch ist der Anteil an verbackenem Material bei den Schuppen deutlich. Zudem lassen sich anhaftende Presslinge durch das Aufwenden geringer Kräfte leicht voneinander lösen. Die anhaftenden NPG-Schuppen können auch durch höhere Kräfte nicht vollständig voneinander gelöst werden.

Zum Vergleich des Auflösungsverhaltens zwischen NPG-Schuppen und -Presslingen wird jeweils eine 25 Gew-% NPG-Lösung in Wasser unter Rühren hergestellt. Das Auflöseverhalten der Briketts ist erstaunlicherweise praktisch unabhängig von der eingesetzten Pressling-Größe. Die Auflösungsgeschwindigkeit der Presslinge beträgt ca. 12:20 min für die 5cm³ bzw. 12:40 min für die 10cm³-Presslinge. NPG-Schuppen lösen sich unter denselben Versuchsbedingungen in ca. 2:53 min auf. Betrachtet man den Unterschied in der zur Auflösung der Schuppen und der Presslinge verfügbaren Oberfläche, so kann man abschätzen, dass die Oberfläche der Schuppen im Vergleich zu den Presslingen um einen Faktor von mindestens 10 höher liegt. Die Schuppen-Oberfläche lässt sich unter der Annahme zylindrischer Teilchen mit der eingesetzten Schuppenhöhe als Höhe und einem Durchmesser entsprechend dem D50-Quantil der Schuppen annähern. Dieser Ansatz lässt den doch erheblichen Anteil sehr kleiner Bruchstücke in den Schuppen unberücksichtigt und führt zu einer sehr konservativen Abschätzung der Schuppen-Oberfläche. Die Presslinge lösen sich im Vergleich zu den Schuppen um einen Faktor 4-5 schlechter, wobei der Unterschied im Faktor bezüglich der verfügbaren Oberfläche auf jeden Fall höher als 10 ist. Insofern lösen sich die Presslinge deutlich besser als erwartet werden konnte. Ohne durch die Theorie gebunden zu sein wird dies auf die spezifische Dichte der Presslinge zurückgeführt, welche angibt, dass kein kompakter NPG-Pressling vorliegt. Die Presslinge enthalten durch den Einsatz von NPG-Schuppen und durch das Anlegen nur geringer Pressdrücke auch noch einen signifikanten Poren- oder Luftanteil im Pressling, welcher anscheinend das Eindiffundieren von Lösemitteln und somit das Auflösen erleichtert.

Einige typische Parameter der durch das erfindungsgemäße Verfahren erhältlichen, erfindungsgemäßen Presslinge ergeben sich wie folgt:

| | 5cm3 | 10cm³ |
|---|---|---|
| Abmessungen L × B × H | 30 × 24 × 10 mm | 33 × 30 × 20 mm |
| Gewicht | 10 g | 13-15 g |
| Oberfläche | 0,0049 m² | 0,0068 m² |
| Oberfläche/Masse | 0,49 m²/kg | 0,49 m²/kg |
| Schüttdichte | 0,535 g/cm³ | 0,531 g/cm³ |
| Dichte | 0,97-0,99 g/cm³ | 0,97-0,99 g/cm³ |
| Feinanteil (< 6mm) | < 1 0% | < 1 0% |
| Masseverlust | 0,22 Gew%/24h 1,6 Gew%/Woche | 0,2 Gew%/24h 1,4 Gew.-%/Woche |

## Patentansprüche

1. Verfahren zur Herstellung von Neopentylglykol-Presslingen, **dadurch gekennzeichnet, dass** das Verfahren mindestens die Verfahrensschritte umfasst:
a) Bereitstellen einer Schüttung aus NPG-Schuppen;
b) Komprimieren der Schüttung in einer Form zur Ausbildung eines Presslings, wobei das Komprimieren bei einem Druck von größer oder gleich 0,5 MPa und kleiner oder gleich 7,5 MPa erfolgt.

2. Verfahren nach Anspruch 1, wobei die NPG-Schuppen eine Schüttdichte von größer oder gleich 0,5 g/cm³ und kleiner oder gleich 0,6 g/cm³ aufweisen.

3. Verfahren nach einem der vorhergehenden Ansprüche, wobei die NPG-Schuppen einen Feinanteil von kleiner oder gleich 6 mm, ermittelt durch Siebung, von größer oder gleich 80 Gew.-% und kleiner oder gleich 90 Gew.-% aufweisen.

4. Verfahren nach einem der vorhergehenden Ansprüche, wobei die NPG-Schuppen eine Dicke von größer oder gleich 0,75 mm und kleiner oder gleich 5 mm aufweisen.

5. Verfahren nach einem der vorhergehenden Ansprüche, wobei die NPG-Schuppen ein D50-Quantil, ermittelt über Siebung, von größer oder gleich 2 mm und kleiner oder gleich 8 mm aufweisen.

6. Verfahren nach einem der vorhergehenden Ansprüche, wobei die NPG-Schuppen ein D95-Quantil, ermittelt über Siebung, von größer oder gleich 7,5 mm und kleiner oder gleich 10 mm aufweisen.

7. Verfahren nach einem der vorhergehenden Ansprüche, wobei das Komprimieren bei einem Druck von größer oder gleich 1,0 MPa und kleiner oder gleich 4 MPa erfolgt.

8. Verfahren nach einem der vorhergehenden Ansprüche, wobei der Verfahrensschritt b) in einem Temperaturbereich von größer oder gleich 5°C und kleiner oder gleich 40°C erfolgt.

9. Verfahren nach einem der vorhergehenden Ansprüche, wobei die NPG-Schuppen einen Wassergehalt von größer oder gleich 0,05 Gew.-% und kleiner oder gleich 3 Gew.-% aufweisen.

10. NPG-Pressling, **dadurch gekennzeichnet, dass** der Pressling eine Dichte von größer oder gleich 0,9 g/cm³ und kleiner oder gleich 1,02 g/cm³ aufweist.

11. NPG-Pressling nach Anspruch 10, wobei der Pressling ein Volumen von größer oder gleich 2,5 cm³ und kleiner oder gleich 15 cm³ aufweist.

12. NPG-Pressling nach Anspruch 10 oder 11, wobei der Pressling eine Dichte von größer oder gleich 0,95 g/cm³ und kleiner oder gleich 1,05 g/cm³ aufweist.

13. NPG-Pressling nach einem der Ansprüche 10-12, wobei der Pressling ein Oberflächen-zu-Massen-Verhältnis von größer oder gleich 0,4 m²/kg und kleiner oder gleich 0,6 m²/kg aufweist.

14. NPG-Pressling nach einem der Ansprüche 10-13, wobei der Pressling einen NPG-Gehalt von größer oder gleich 98 Gew-% aufweist.

15. NPG-Pressling nach einem der Ansprüche 10-14, wobei der Pressling eine Druckfestigkeit von größer oder gleich 80 N und kleiner oder gleich 400 N aufweist.
